# EUROPÄISCHE PATENTSCHRIFT

(11) **EP 0 631 789 B1**
(45) Veröffentlichungstag und Bekanntmachung des Hinweises auf die Patenterteilung: **01.09.1999**
(21) Anmeldenummer: 94109061.5
(22) Anmeldetag: 14.06.1994
(51) Int. Cl.: A61M 1/16

(54) **Vorrichtung zum Gasaustausch, insbesondere Oxygenierung in Blut**
Device for gaseous exchange especially for oxygenating blood
Dispositif pour l'échange gazeux en particulier pour l'oxygénation du sang

(30) Priorität: 18.06.1993 DE 4320198
(43) Veröffentlichungstag der Anmeldung: 04.01.1995
(73) Patentinhaber: Fresenius Medical Care Deutschland GmbH, 61350 Bad Homburg v.d.H. (DE)
(72) Erfinder: Polaschegg, Hans-Dietrich, Dr., D-61440 Oberursel (DE); Lewinski, Hans Helmut, Dr., D-60385 Frankfurt (DE); Steinbach, Bernd, Dr., D-61348 Bad Homburg (DE)
(74) Vertreter: Luderschmidt, Schüler & Partner GbR

(56) Entgegenhaltungen:
- EP-A- 0 323 341
- WO-A-84/00015
- WO-A-93/11807
- DE-A- 3 129 064
- DE-C- 4 028 311

## Beschreibung

Die Erfindung betrifft eine Vorrichtung zum Gasaustausch, insbesondere Oxygenierung in Blut mit einem Gasaustauscher, der durch eine semipermeable Membran in eine Blutkammer und eine Gasaustauschkammer geteilt ist, wobei durch die Blutkammer ein extrakorporaler Blutweg und durch die Gasaustauschkammer ein Gasweg gelegt sind.

Beim Gasaustausch zwischen einer flüssigen Phase und einer gasförmigen Phase mittels mikroporöser Membranen werden üblicherweise hydrophobe Membranen eingesetzt. Dies ist beispielsweise bei der extrakorporalen Membranoxygenierung (ECMO) der Fall, bei der im extrakorporalen Kreislauf, beispielsweise bei der Durchführung einer Operation am offenen Herzen Blut mit Sauerstoff versorgt wird.

ECMO-Vorrichtungen sind beispielsweise im ASAIO, Vol. XXXIV (1988), Seite 978-985, beschrieben. Desgleichen ist ein hydrophober Oxygenator in der DE-A-31 29 064 beschrieben, die den Einsatz von hydrophoben Membranmaterialien, beispielsweise in Form von Hohlfasern zur Oxygenierung vorschlägt. Dabei wird dann auf der einen Seite einer hydrophoben Membran Blut im extrakorporalen Kreislauf vorbeigeführt, während an der anderen Seite der Membran im Gegenstrom Sauerstoff zugeführt wird, so daß über die Poren der Membran hinweg ein CO₂/O₂-Austausch erfolgen kann.

Aus der WO 84/00015 sind hydrophobe Membranen bekannt, die üblicherweise auf dem Blutoxygenierungssektor eingesetzt werden. Um diese hydrophoben Membranen für die Plasmapherese einsetzbar zu machen, werden diese hydrophiliert, um Plasmafraktionen aus dem Blut abzutrennen.

Es werden üblicherweise zwei Typen von hydrophoben Membranen eingesetzt, nämlich Membranen, die aus einem per se hydrophoben Material, beispielsweise Polypropylen, bestehen und Membranen, deren Oberfläche mit einem Hydrophobierungsmittel, beispielsweise Silikon hydrophob gemacht wurden.

Hydrophobe Membranen aus hydrophoben Materialien, wie PP, weisen vergleichsweise große Poren mehrere 100 bis 1000 nm auf und liegen in Form von mehreren 1000 Hohlfäden vor, was in einer aktiven Oberfläche bis zu 6 m² resultiert. Das Blut fließt dabei entweder in den Hohlfäden oder aber auf der Außenseite des Hohlfadens, während das auszutauschende Gas auf der gegenüberliegenden Seite im Gegenstrom hierzu fließt. Diese Membranen werden üblicherweise in Herz-Lungen-Maschinen eingesetzt.

Hydrophobierte Membranen bestehen andererseits aus einer dünnen Schicht Silikon auf einer porösen Trägerstruktur und werden - wie nachstehend erläutert - für die Langzeit-ECMO-Behandlung eingesetzt.

Zwar sind hydrophobe Membranen im Vergleich zu silikonisierten Membranen effektiver, was die Diffusion der Gase in den gasgefüllten Poren betrifft, da die Diffusion in Poren wesentlich schneller abläuft als durch flüssige Grenzflächen.

Andererseits haben jedoch Membranen aus hydrophobem porösen Material einen signifikanten Nachteil in der Langzeittherapie, d.h. in der Therapie, die sich über einen Zeitraum von mehr als 6 Stunden erstreckt. Dieser Nachteil besteht im Lecken der Membran, da sich die Poren trotz ihrer hydrophoben Struktur mit wässrigen Plasmabestandteilen füllen, was zu einer Hydrophilierung bzw. Benetzung der Oberfläche der Membran führt. Nachdem die hydrophoben Membranen mit einem positiven Transmembrandruck (TMP) von der Blut- zur Gasseite gesehen eingesetzt werden, führt die Hydrophilierung einerseits zum freien Fließen des Plasmas von der Blut- zur Gasseite, so daß Plasma den Oxygenator in flüssiger Form oder als Schaum verlassen kann, und andererseits zu einer Behinderung der Gasdiffusion durch die Verstopfung der Membranen mit Plasma, so daß die Effizienz der Vorrichtung drastisch vermindert wird. In einem solchen Fall muß also der Oxygenator ausgetauscht werden.

Silikonmembranen sind andererseits weniger wirksam, verhindern jedoch ein Durchschlagen von Plasma und werden somit in der Langzeit-ECMO eingesetzt.

In ähnlicher Weise wurden hydrophobe Membranen bei der extrakorporalen Entfernung von CO₂ (ECCO₂R) aus Blut eingesetzt. Eine solche Vorrichtung ist beispielsweise in der DE-A-40 28 311 dargestellt.

Es wurde bereits vorgeschlagen, auch hydrophile Membranen für ECMO einzusetzen, beispielsweise im IEEE 13 (1991), Seite 1557-1559. Bei der dort vorgeschlagenen Anordnung erfolgt die Oxygenierung in mehreren Kreisläufen, die jeweils mit semipermeablen Membranen voneinander getrennt sind. Die unmittelbare Oxygenierung erfolgt zunächst mit dem üblichen hydrophoben Oxygenator, auf dessen einer Seite Sauerstoff entlangläuft und dessen andere Seite mit wässriger Flüssigkeit beaufschlagt wird. Diese wässrige Flüssigkeit nimmt Sauerstoft in einem bestimmten Partialdruck auf und gibt diesen entlang einer hydrophilen Membran an einen Blutkreislauf ab, d.h., es erfolgt ein Austausch von Flüssigkeit unterschiedlichen Gasgehalts entlang der Oberfläche der hydrophilen Membran. Eine solche Vorrichtung bedient sich also der Klassischen hydrophoben Membran zum Transfer von gasförmigem Sauerstoff in eine wässrige Flüssigkeit.

Der Erfindung liegt die Aufgabe zugrunde, eine Vorrichtung zum Gasaustausch im Blut zur Verfügung zu stellen, die einerseits eine hohe Austauschleistung aufweist und andererseits auch im Langzeitbetrieb eingesetzt werden kann.

Die Lösung der Aufgabe gelingt dadurch, daß die Membran hydrophiliert ist und im Betrieb mit Wasser benetzt ist und der Gasweg mit sauerstoffhaltigem Gas unter positivem Transmembrandruck von der Gas- zur Blutseite in Betrieb beaufschlagt ist.

Unter "hydrophiler Membran" werden sämtliche mit Wasser benetzbaren Membranen verstanden, also zunächst Membranen, die aus einem eine oder mehrere Komponenten enthaltenden hydrophilen Material, wie regenerierter Cellulose oder Polysulfon, das mit Polyvinylpyrrolidon (PVP) bestimmten Mengen hydrophiliert ist, bestehen. Ein Filter mit dem letztgenannten Material ist beispielsweise von der Anmelderin mit der Bezeichnung PS 400 im Handel und ist im übrigen in der DE-A-34 26 331 beschrieben, auf deren Offenbarung inhaltlich voll bezug genommen wird. Weitere Membranmaterialien sind Polyacrylnitril, Cuprophan, CA und dergleichen. Festzustellen ist, daß diese Membranen bereits seit langen Jahren für die Hämodialyse eingesetzt werden und sich dort bewährt haben.

Neben Membranen aus bereits hydrophilem Material können auch hydrophobe Membranen eingesetzt werden, sofern sie mit auswaschbaren Hydrophilierungsmitteln, wie Myristylalkohol oder mit einem Wasser/Ethanol-Gemisch hydrophil gemacht werden. Setzt man beispielsweise das letztgenannte Gemisch ein, so kann eine mit diesem Gemisch gefüllte Membran direkt durch Einleiten von Wasser oder einer wässrigen Elektrolytlösung benetzt werden, d.h., sämtliche Poren sind dann mit der wässrigen Flüssigkeit gefüllt. Eine weitere Hydrophilierungsmethode mit Hilfe von Natriumacetat ist beispielsweise in der DE-A-30 43 073 beschrieben.

Erfindungswesentlich ist es also, daß die Poren der Membran mit wässriger Flüssigkeit gefüllt sind.

Erfindungsgemäß werden solche Membranen eingesetzt, die aus hydrophilem oder durch hydrophile Zusätze hydrophil gemachten Material bestehen.

Hydrophile Membranen, wie sie beispielsweise in der DE-A-34 26 331 beschrieben sind, weisen üblicherweise eine asymmetrische Gestalt auf, d.h. sie bestehen aus einer relativ dünnen Haut (Skin) von etwa 1 µm, die von einer relativ grobporigen Stützstruktur gestützt ist. Beide Strukturen bestehen aus ein und demselben Material, wobei die dünnporige Membran Poren relativ geringen Durchmessers (zwischen 2- und 20 nm) aufweist. Durch diese Poren erfolgt der Gasaustausch über die in der Pore befindliche Flüssigkeitssäule, wobei sich das im Gasstrom befindliche Molekül an der Gas-Flüssigkeitsgrenzfläche mit dem in der Flüssigkeit physikalisch gelösten Gasmolekül im Gleichgewicht befindet. Insofern dient der dort befindliche Flüssigkeitsfilm als Barriere für Gase ähnlich der Silikonmembran mit dem Unterschied, daß die Austauschleistung wesentlich größer ist, da die Diffusionskonstante von gelöstem Sauerstoff in Wasser im Vergleich zu Silikon wesentlich höher ist.

Die in einer Pore befindliche Flüssigkeit kann in Abhängigkeit vom Porendurchmesser und von der Länge der Pore bei einem bestimmten Druck aus der Pore dadurch ausgepreßt werden, daß auf der anderen Seite der Membran ein Gas mit einem bestimmten Überdruck angelegt wird. Sobald der Druck einen bestimmten Wert übersteigt, wird die Flüssigkeit komplett aus der Pore gepreßt, so daß Gasblasen in der Flüssigkeit sichtbar sind (sogenannter bubble-point-Druck). Dieses Verfahren wird verwendet, um hydrophile Membranen, beispielsweise bei Hämodialysatoren zur Erkennung von Defekten (große Löcher oder gebrochene Kapillaren) zu erkennen. Üblicherweise liegt der bubble-point von solchen Membranen in Wasser bei einem Wert oberhalb 10 bar, so daß mit Wasser benetzte Membranen bis zu 10 bar dicht sind und mit Gas beaufschlagt werden können, ohne daß ein Durchschlagen von Gas zu befürchten ist.

Nun hängt aber der in einer Flüssigkeit vorliegende Gaspartialdruck, der mit der in der Flüssigkeit gelösten Gasmenge korreliert ist, von dem angelegten Außendruck und/oder dem Gaspartialdruck der jeweiligen Gaskomponente in dem Gasgemisch (wie der Komponente Sauerstoff in Luft) ab mit der Folge, daß über den angelegten Außendruck die dem Blut zuzuführende Gasmenge eingestellt werden kann. Infolgedessen kann die Sauerstoffkonzentration durch die Wahl des Luft/Sauerstoffgemisches oder des Druckes auf dieses Gemisch eingestellt werden.

Wie bereits vorstehend erwähnt, liegen die Membranen vorteilhafterweise in Form von Hohlfäden vor. Andererseits können jedoch aber auch Flachmembranen eingesetzt werden. Die Hohlfäden besitzen einen Außendurchmesser von 100-400 µm, vorteilhafterweise etwa 200 µm, sowie einen Innendurchmesser von etwa 80-320 µm. Die Wandstärke liegt in einem Bereich von 10-60 µm.

Üblicherweise enthält eine erfindungsgemäße Gasaustauschvorrichtung, insbesondere Oxygenator bis zu 10000 Hohlfäden je Einheit, so daß die gesamte Membranoberfläche bis zu 10 m² betragen kann.

In Betrieb dient die mit Wasser benetzte Membran als Flüssigmembran, durch die der Austausch bzw. Transfer der Gase von dem einen Medium Gas zum anderen Medium Flüssigkeit erfolgt. Demzufolge wird im Blut befindliches freies CO₂ an der Membranoberfläche ausgetauscht gegen das von der anderen Membranseite her transferierte Gas, das üblicherweise aus Luft besteht, die mit Sauerstoff in einem vorbestimmten Verhältnis angereichert ist. Vorteilhafterweise wird das Gas mit einem positiven TMP (von der Gasseite zur Blutseite gesehen) gegen die flüssige Grenzfläche gedrückt, in der es sich physikalisch mit dem entsprechenden Partialdruck löst. Ausgeschlossen ist dabei ein Transfer von gasförmigen Bestandteilen aufgrund der Tatsache, daß der bubble-point bei dem gewählten absoluten Überdruck nicht überschritten wird. Insofern besteht im Gegensatz zu hydrophoben Membranen bei den mit Wasser benetzten hydrophilen Membranen nicht das Risiko, daß direkt Gasblasen in die Blutbahn durch die Poren der Membran gefördert werden und somit eine Luftembolie erzeugen könnten.

Letzteres kann nur dann geschehen, wenn es zu einer Ruptur in der Membranoberfläche kommt, so daß Luft in den Blutstrom eintreten kann. Insofern sind zweckmäßigerweise Sicherheitseinrichtungen in Form von Luftdetektoren stromab der Gasaustauschvorrichtung vorgesehen, um sicher die Entstehung von Gasblasen zu erkennen.

Eine solche Freisetzung von Luftblasen kann im übrigen auch dann erfolgen, wenn eine Übersättigung des Blutes mit Sauerstoff enthaltenem Gas aufgrund eines zu hohen TMP gegenüber Umgebungsdruck entsteht, so daß auch hier zweckmäßigerweise ein Luftsensor stromab der Gasaustauschvorrichtung vorgesehen ist. Eine solche Übersättigung hängt - wie festgestellt - von einem zu hohen TMP ab, der relativ zu den beiden Medien eingestellt wird, während die Übersättigung - absolut zum Umgebungsdruck betrachtet - auftritt.

Gemäß einem weiteren unabhängigen Erfindungsgedanken wird die Austauschvorrichtung stromauf einer Pumpe, insbesondere der Blutpumpe, angeordnet, so daß sich hierdurch zwangsläufig ein Unterdruck im Ansaugbereich der Pumpe ergibt. Wird hier eine Oxygenierung vorgenommen, so stellt sich entsprechend dem positiven TMP eine bestimmte Sauerstoff/Luftkonzentration im Blut ein, die im Unterdruckbereich übersättigt sein kann. Wird jedoch das Blut auf die Druckseite der Pumpe überführt, ändert sich durch die Druckerhöhung zwangsläufig die Löslichkeit des Gases in Blut, d.h., es löst sich unter dem erhöhten Druck mehr Gas im Blut als bei dem niedrigeren Ansaugdruck. So kann eine mit Gas übersättigte Lösung des Blutes durch Druckerhöhung in einen nicht übersättigten Bereich überführt werden, so daß die Bildung von Gasblasen mit einer solchen Vorrichtung wirksam unterdrückt wird.

Andererseits kann anstelle der Anordnung stromauf der Blutpumpe auch eine Ausführungsform stromab der Blutpumpe aussehen, bei der die Gasaustauschvorrichtung zwischen einer Strömungsdrosseleinrichtung stromauf und einer weiteren Pumpe stromab angeordnet ist, so daß hierdurch ein künstlicher Unterdruckbereich geschaffen wird.

Gemäß einer ersten Ausführungsform ist nur eine Gasaustauschvorrichtung im extrakorporalen Kreislauf vorgesehen, die auf der einen Seite der Membran mit zu oxygenierendem Blut und auf der anderen Seite mit einem Gasstrom mit einem vorbestimmten O₂-Partialdruck (Konzentration), der jedoch bei der Oxygenierung verändert werden kann, beaufschlagt wird. Der Gasstrom wird dabei mit einem vorbestimmten oder geregelten Überdruck der mit Wasser benetzten Membran im Gegenstrom zugeführt, so daß CO₂ und das Sauerstoff/Luftgemisch wechselweise von der einen Seite zur anderen Seite der Membran transferiert werden.

Bei dieser Ausführungsform kann die Zuführung des Sauerstoff/Luftgemisches sowohl gesteuert als auch geregelt erfolgen. Im Steuerungsfall wird vorher die Strömungsrate des Blutes und die Strömungsrate sowie die Zusammensetzung des Sauerstoff/Luftgemisches einschließlich des Transmembrandrucks ermittelt, wobei dann vorbestimmte Druck- und Strömungswerte eingehalten werden. Sofern eine Regelung erfolgt, ist stromab des Gasaustauschers ein O₂-Sensor im Blutkreislauf vorgesehen, mit dem die aktuelle O₂-Konzentration festgestellt wird. Der ermittelte Wert dient dann als Ist-Wert, mit dem dann ein Vergleich mit einem Soll-Wert vorgenommen wird, dessen Ergebnis zu einer Änderung der Zusammensetzung oder des Überdrucks des Gasgemisches eingesetzt werden kann. Die Druckveränderung kann erfindungsgemäß an einem Druckregelventil oder einem Gasdrosselorgan proportional oder intermittierend erfolgen. Während bei der proportionalen Druckveränderung stetig der Druck entsprechend einem bestimmten Wert konstant gehalten wird, erfolgt bei der intermittierenden Betriebsweise ein taktweises Öffnen und Schließen des Ventils, wobei der Druck entweder durch das Verhältnis der Öffnungszeiten des Ventils zu den Verschlußzeiten oder durch die Häufigung der Öffnung bei konstanter Öffnungszeit konstant gehalten wird.

Die Regelung des Drucks erfordert einen Sensor im Gasbereich sowie eine Aussteuerungseinrichtung des Regelventils.

Gemäß einer weiteren Ausführungsform sind im extrakorporalen Blutkreislauf zwei Gasaustauscher vorgesehen, von denen der eine zum CO₂-Austausch herangezogen wird, während der nachgeschaltete andere Austauscher zur reinen Oxygenierung dient. Vorteilhafterweise wird der CO₂-Austauscher mit Luft beaufschlagt, wobei ein TMP von etwa 0 eingesetzt wird. Die Luft wird dabei stetig der Membran zugeführt und verläßt den Gasaustauscher auf der gegenüberliegenden Seite. Vorteilhafterweise wird der Partialdruck von CO₂ stromab oder stromauf des Austauschers bestimmt. Das Signal kann auf eine Steuereinheit gegeben werden, die den Fluß der Luft steuert, um einen bestimmten CO₂-Gehalt stromab des Filters einzustellen.

Der zweite Gasaustauscher dient zur reinen Oxygenierung und kann einen Kleineren Oberflächenbereich als der erste Gasaustauscher aufweisen. Vorzugsweise wird Sauerstoff im dead-end-Betrieb dem Oxygenator zugeführt, d.h., dieser Oxygenator weist keinen Gasauslaß auf. Der Sauerstoffluß wird entweder gesteuert oder geregelt eingestellt. Im geregelten Fall wird der Sauerstoff-Partialdruck und/oder die Sauerstoffsättigung mit Hilfe eines Sensors stromab ermittelt und das Signal wiederum auf eine Reglereinheit gegeben, die den Sauerstoffluß entsprechend einstellt. Der erhaltene Sauerstoff-Partialdruck oder die Übersättigung stellt sich entsprechend einem vom Verbraucher definierten Wert ein (beispielsweise ein Sauerstoff-Partialdruck von 0,25 bar oder eine Sauerstoffsättigung von 99 %).

Andererseits kann jedoch aber auch das gesamte System gesteuert im Proportionalbetrieb betrieben werden, bei dem der Sauerstoffluß proportional zum Blutfluß folgt. In einem solchen Fall werden die CO₂/O₂-Austauschleistung der beiden Austauscher in Relation zum Blutfluß gebracht und somit gesteuert betrieben.

Verglichen zum konventionellen Oxygenierungssystem, bei dem hydrophobe Oxygenatoren eingesetzt werden, hat das erfindungsgemäße System folgende Vorteile:
Es können Standard- oder etwas modifizierte Dialysatoren eingesetzten werden, die Membranen aus vergleichsweise biokompatiblen Materialien, wie Polysulfon, besitzen. Erheblich sind dabei die Kosteneinsparungen, da Dialysatoren unter 10 % der Kosten eines Membranoxygenators liegen.

Beim Einsatz von zwei Austauschern ist kein Gasmischer mehr notwendig und der Sauerstofftransfer kann unabhängig von dem zu entfernenden CO₂ eingestellt werden. Hierdurch wird eine einfache automatische Steuerung möglich, die nur von einem einzigen Parameter abhängt, nämlich dem Sauerstoff- oder CO₂-Gehalt, der jeweils mit entsprechenden Sensoren bestimmt werden kann.

Der Sauerstoffverbrauch ist auf den Sauerstoff begrenzt, der vom Patienten benötigt wird.

Schließlich kann der CO₂-Filter auch zur Entfernung von Flüssigkeit eingesetzt werden, ohne daß hierdurch die CO₂-Entfernung wesentlich beeinträchtigt wird.

Mit dem erfindungsgemäßen Gasaustauscher soll die Summe der Partialdrücke im Blut, die den Oxygenator verlassen, den Atmosphärendruck erreichen. Dieser darf - wie vorstehend erläutert - nicht wesentlich überschritten werden, da ansonsten die Gefahr der Luftembolie besteht.

Die Zeichnung erläutert die Erfindung.

Es zeigen
**Figur 1** eine erste Ausführungsform einer Gasaustauschvorrichtung in schematischer Darstellung,
**Figur 2** eine zweite Ausführungsform einer Gasaustauschvorrichtung in schematischer Form und
**Figur 3** eine dritte Ausführungsform einer Gasaustauschvorrichtung mit einem CO₂-Austauscher und einem Oxygenator in schematischer Form.

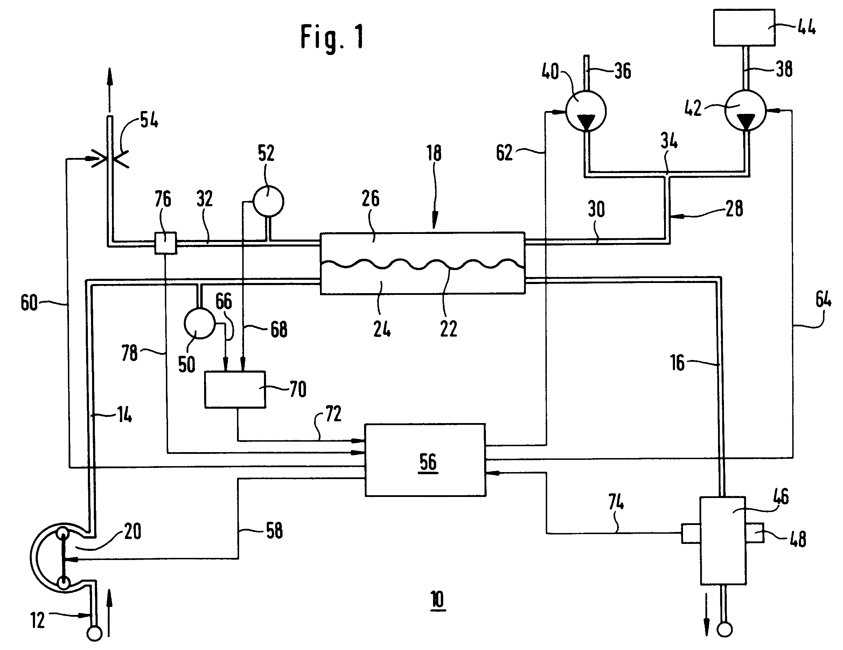

In Figur 1 ist mit 10 eine Gasaustauschvorrichtung schematisch dargestellt.

Diese Gasaustauschvorrichtung weist einen extrakorporalen Blutkreislauf 12 auf, der in eine Zuleitung 14 und eine Ableitung 16 durch eine Gasaustauscheinheit 18 geteilt ist.

In die Zuleitung ist eine Blutpumpe 20 eingeschaltet.

Die Gausaustauscheinheit 18 ist durch eine semipermeable Membran 22 in eine Blutkammer 24 und eine Gasaustauschkammer 26 geteilt. Durch die Blutkammer 24 ist der extrakorporale Kreislauf 12 gelegt bzw. an den Eingang der Blutkammer 24 ist die Zuleitung 14 und an den Ausgang die Ableitung 16 angeschlossen.

Durch die Gasaustauschkammer ist eine Gasleitung 28 gelegt, die in eine Gaszuleitung 30 und eine Gasableitung 32 geteilt ist.

Das Ende der Gaszuleitung 30 mündet in einen Gasmischpunkt 34, an den eine Luftzuleitung 36 und eine Sauerstoffzuleitung 38 angeschlossen sind. In die beiden Zuleitungen 36 und 38 sind jeweils Gasförderorgane 40 und 42 eingeschaltet. Während die Luftzuleitung 36 an die Umgebungsluft angeschlossen ist, ist die Sauerstoffzuleitung 38 an eine Sauerstoffquelle 44 angeschlossen.

In die Ableitung 16 ist weiterhin eine Tropfkammer 46 eingeschaltet, die einen Gassicherheitssensor 48 aufweist.

Die Zuleitung 14 weist benachbart zur Blutkammer 24 einen ersten Drucksensor 50 zur Bestimmung des im Blut vorliegenden Drucks auf.

Desgleichen ist in der Gasableitung 32 ein zweiter Drucksensor 52 vorgesehen, mit dem der in der Gasleitung vorliegende Gasdruck bestimmt werden kann.

Schließlich ist am Ende der Gasableitung 32 eine Klemmenanordnung 54 vorgesehen, mit der der Querschnitt der Gasableitung 32 verändert werden kann.

Die Gasaustauschvorrichtung 10 wird mittels einer Steuereinheit 56 gesteuert. Hierzu ist die Steuereinheit 56 über Steuerleitungen 58 - 64 mit der Blutpumpe 20, der Klemmenanordnung 54, dem ersten Gasförderorgan 40 und dem zweiten Gasförderorgan 42 verbunden.

Die Drucksensoren 50 und 52 geben über Signalleitungen 66 und 68 Signale an eine TMP-Ermittlungseinheit 70 ab, deren Signal über die Signalleitung 72 auf die Steuereinheit 56 geschaltet ist. Weiterhin ist die Steuereinheit 56 über eine Signalleitung 74 mit dem Gassicherheitssensor 48 verbunden.

Schließlich können in der Gasableitung 32 ein CO₂-Sensor 76 vorgesehen sein, der über eine Signalleitung 78 mit der Steuereinheit 56 verbunden ist.

Die Gasaustauschvorrichtung 10 wird folgendermaßen betrieben.

Der extrakoporale Kreislauf 12 wird vor dem Anschluß an einen Patienten mit einer sterilen physiologischen Kochsalzlösung gefüllt, so daß es - wie eingangs erläutert - zu einer Benetzung der hydrophilen Membran 22 kommt. Anschließend wird der extrakorporale Kreislauf 12 an den Patienten angeschlossen und die Blutpumpe 20 mit einer vorbestimmten Geschwindigkeit von der Steuereinheit 56 in Betrieb genommen. Hier stellt sich dann ein positiver Druck in der Zuleitung 14 stromab der Blutpumpe 20 und stromauf der Gasaustauscheinheit 18 ein, die von dem Drucksensor 50 fortlaufend bestimmt und an die TMP-Ermittlungseinheit 70 abgegeben wird.

Die Steuereinheit 50 nimmt weiterhin das erste Gasförderorgan 40 zur Zuführung von Luft in vorbestimmter Weise in Betrieb und führt diese dem Gasmischpunkt 34 zu. Desweiteren wird die zweite Gasfördereinheit 42 in vorbestimmter Weise in Betrieb genommen und fördert eine vorbestimmte Menge Sauerstoff zum Gasmischpunkt 34, an den ein Sauerstoff-Luftgemisch mit vorbestimmter Zusammensetzung gemischt und über die Gaszuleitung 30 der Gasaustauschkammer 26 zugeführt wird.

Der Gasdruck, der an die Membran 22 angelegt wird, wird mittels der steuerbaren Klemmenanordnung 54 eingestellt und mit dem Drucksensor 52 ermittelt, der sein Signal ebenfalls an die TMP-Ermittlungseinheit 70 abgibt, in der der Transmembrandruck (TMP) ermittelt und an die Steuereinheit 56 gegeben wird.

Gemäß einem vorbestimmten Programm, das in der Steuereinheit 56 abgelegt ist, erfolgt eine Zuführung von Sauerstoff von ca. 50 Nml/min, wobei zugleich im wesentlichen gleichen Mengen CO₂ aus dem Blut abgefördert werden.

Dabei kann der CO₂-Gehalt über die Gasförderrate der Gasförderorgane 40 und 42 und den CO₂-Sensor 76 gegebenenfalls ermittelt und zu Regelzwecken eingestellt werden.

Mit der Gasaustauscheinheit 10 erfolgt also ein kombinierter Gasaustausch von CO₂ und O₂ entsprechend den vorgegebenen Partialdrücken im Gasgemisch bzw. dem angelegten TMP. Dieser TMP wird letztlich eingestellt, einerseits über die Blutpumpe 20 und andererseits über die Klemmenanordnung 54 in Abhängigkeit von dem Strömungswiderstand des Gasaustauschers 18 bzw. den Förderraten der Gasförderorgane 40 und 42. Ausgangs der Blutkammer 24 wird dann ein an CO₂ abgereichertes und ein an O₂ angereichertes Blut erhalten.

Wie bereits vorstehend festgestellt, arbeitet die in Figur 1 dargestellte Vorrichtung 10 stromab der Blutpumpe 20 im Überdruckbereich, d.h. am Eingang der Blutkammer 24 wird Blut mit Überdruck und am Ausgang der Blutkammer üblicherweise Blut mit Umgebungsdruck gefördert. Nachdem sich mehr Gas im Blut bei Überdruck löst als bei Normaldruck, muß erfindungsgemäß darauf geachtet werden, daß es nicht zu einer Übersättigung des Blutes bei der Oxygenierung kommt, was zur Bildung von Gasblasen ausgangs der Blutkammer 24 führen könnte. Zur Vermeidung von Alarmsituationen ist daher der TMP so einzustellen, daß solche Überdruckverhältnisse nicht entstehen.

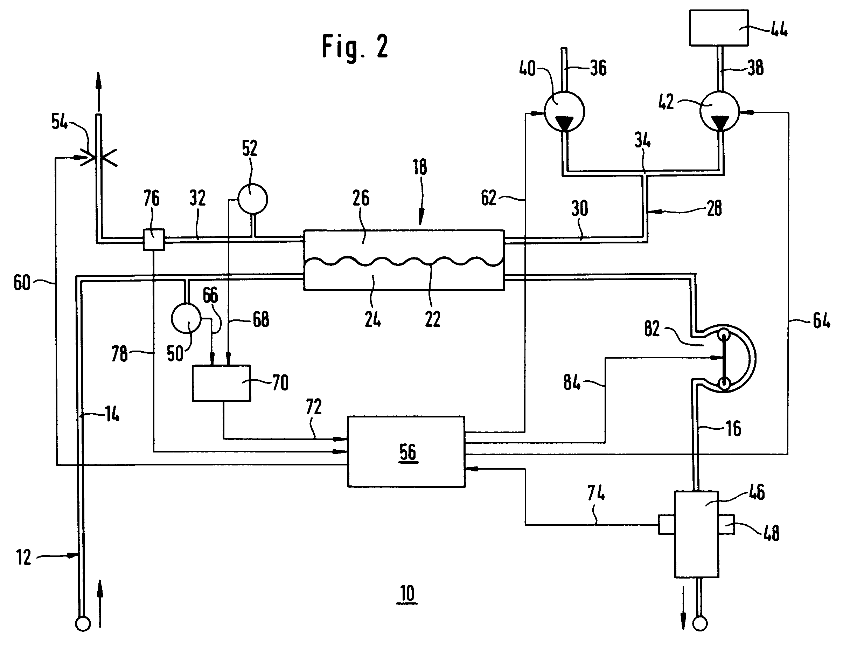

Die in Figur 2 gezeigte Ausführungsform zeigt eine weitere Gasaustauschvorrichtung 10, die in ihrem Aufbau bis auf die Anordnung der Blutpumpe 82 der Vorrichtung 10 gemäß Figur 1 identisch gleicht, so daß für gleiche Teile die gleichen Bezugszeichen wie in Figur 1 eingesetzt werden.

Die Blutpumpe 82 ist stromab der Gasaustauscheinheit 18 in der Ableitung 16 jedoch stromauf der Druckkammer 46 angeordnet und über eine Steuerleitung 84 mit der Steuereinheit 56 verbunden. Insofern befindet sich die Gasauscheinheit 18 im Ansaug- oder Unterdruckbereich der Blutpumpe 82 mit der Folge, daß stromab der Blutpumpe 82 das mit Gas angreicherte Blut auf den Umgebungsdruck angehoben wird, so daß hierdurch zwangsläufig das vorstehend geschilderte Übersättigungsproblem entfällt.

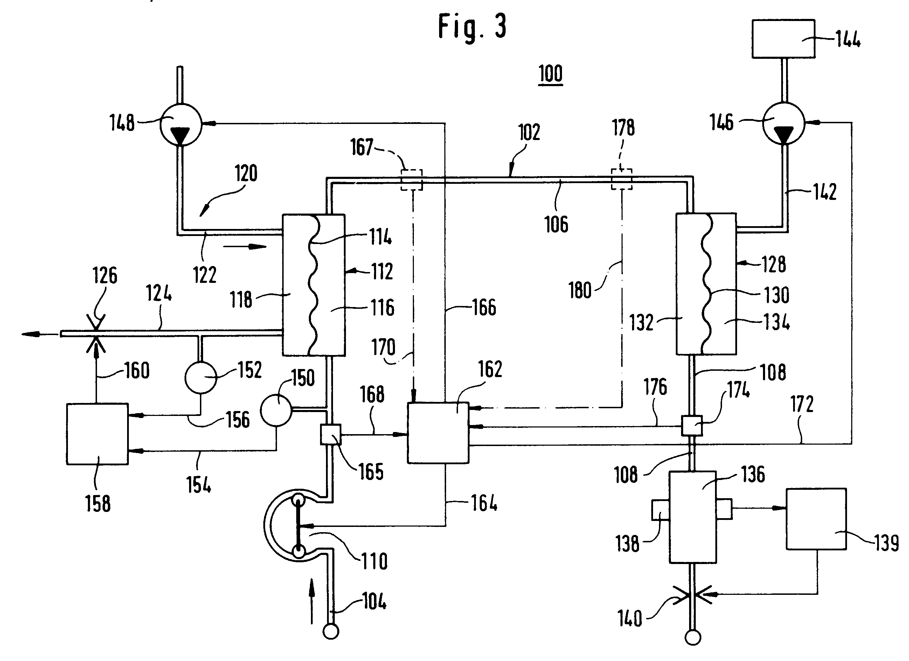

In Figur 3 ist eine dritte Ausführungsform einer Gasaustauschvorrichtung 100 gezeigt, die einen extrakorporalen Blutkreislauf 102 aufweist, der in eine Zuleitung 104, eine Übergangsleitung 106 und eine Ableitung 108 geteilt ist.

In die Zuleitung 104 ist eine Blutpumpe 110 eingeschaltet. Das eine Ende der Zuleitung 104 kann mit einem nicht gezeigten Patienten verbunden werden, während das andere Ende mit einem CO₂-Austauscher verbunden ist, der eine semipermeable hydrophilierte Membran 114 aufweist, die den CO₂-Austauscher in eine Blutkammer 116 und eine Entgasungskammer 118 teilt. Durch die Entgasungskammer 118 ist ein Entgasungsweg 120 gelegt, der in eine Entgasungszuleitung 122 und eine Entgasungsableitung 124 geteilt ist.

Am Ausgang der Blutkammer 116 geht die Übergangsleitung 106 ab und ist an ihrem anderen Ende mit einem Oxygenator 128 verbunden, der ebenfalls durch eine hydrophilierte semipermeable Membran 130 in eine Blutkammer 132 und eine Oxygenierungskammer 134 geteilt ist. Die Übergangsleitung 106 mündet dabei in die Blutkammer 132. Von dieser Blutkammer geht auf der anderen Seite die Ableitung 108 ab, in die eine Tropfkammer 136 eingeschaltet ist, die mit einem Gassicherheitssensor 138 ausgerüstet ist.

Stromab der Tropfkammer 136 ist eine Sicherheitsklemme 140 an der Ableitung 108 vorgesehen.

In die Oxygenierungskammer 134 mündet eine Oxygenierungsleitung 142, die vorteilhafterweise keine Ablaßleitung aufweist und somit im dead-end-Betrieb betrieben wird. Die Oxygenierungsleitung 142 ist mit einer Sauerstoffquelle 144 verbunden und weist ein Sauerstoff-Förderorgan 146 auf.

Ein ähnliches Luftförderorgan 148 ist am Eingang der Entgasungsleitung 120 versehen, über die Luft gefördert wird. Diese Förderorgane können als Pumpen oder aber, sofern das zu fördernde Gas bereits mit Überdruck angeliefert wird, als Gasdruckregelventile bzw. Gasflußregelventile ausgebildet sein.

Desweiteren ist am Ausgang der Entgasungsableitung 124 ein Gasdrosselorgan 126 vorgesehen, mit dem der Entgasungsdruck in dem Entgasungsweg 120 steuerbar ist.

Um den Transmembrandruck über die Membran 114 festzustellen, ist stromauf des CO₂-Austauschers in der Blutzuleitung 104 ein erster Drucksensor 150 vorgesehen, um den Blutdruck in der Zuleitung zu bestimmen.

Ein zweiter Drucksensor 152 ist in der Entgasungsableitung 124 stromauf der Drossel 126 vorgesehen. Beide Drucksensoren 150 und 152 geben über Steuerleitungen 154 und 156 ihr Signal an eine Steuereinheit 158 ab, die den Transmembrandruck bestimmt und den ermittelten Transmembrandruck mit einem voreingestellten Wert vergleicht und über die Steuerleitung 160 das Gasdrosselorgan 126 entsprechend verstellt. Andererseits kann jedoch aber auch die Förderrate der Blutpumpe 110 entsprechend angepaßt werden.

Üblicherweise wird der Transmembrandruck im CO₂-Austauscher 112 so eingestellt, daß die beiden Teildrücke sich aufheben, so daß der Transmembrandruck etwa 0 ist. Gegebenenfalls kann auf der Blutseite ein Überdruck vorliegen, sofern überschüssige Flüssigkeit entfernt werden soll, die dann durch die als Filtratleitung dienende Gasabzugsleitung 124 entfernt wird.

Desweiteren ist eine Hauptsteuereinheit 162 vorgesehen, die über die Steuerleitung 164 die Blutpumpe 110 sowie über eine Steuerleitung 166 das Luftförderorgan 148 entsprechend einer vorbestimmten CO₂-Entfernungsrate steuert.

Vorteilhafterweise kann ein CO₂-Sensor entweder stromauf oder stromab des CO₂-Austauschers 112 in der Zuleitung 104 oder in der Übergangsleitung 106 als Sensor 165 bzw. 167 vorgesehen sein, die über Signalleitungen 168 bzw. 170 mit der Hauptsteuereinheit 162 verbunden sind. Mittels eines der Signale der CO₂-Sensoren 165, 167 läßt sich dann bei bekanntem Transmembrandruck der zuzuführende Luftstrom an dem Luftförderorgan 148 bzw. an der Drosseleinheit 126 einstellen.

Die Hauptsteuereinheit 162 ist weiterhin über eine Steuerleitung 172 mit dem Sauerstoff-Förderorgan 146 verbunden und stellt diese auf einen vorbestimmten Wert ein.

Vorteilhafterweise ist stromab des Oxygenators 128 in der Ableitung 108 ein Sauerstoffsensor 174 vorgesehen, dessen Signal über die Signalleitung 176 auf die Hauptsteuereinheit 162 geschaltet ist. Wird mit dem Sauerstoffsensor 174 der aktuelle Sauerstoffgehalt im Blut ermittelt, so kann dieses Ist-Signal mit einem Soll-Wert in der Hauptsteuereinheit 162 verglichen werden, die dann das O₂-Förderorgan entsprechend dem durchgeführten Vergleichsergebnis regelt.

Der Luftdetektor 138 ist über eine Sicherheitssteuerung 139 verbunden, die bei Detektion von Luft die Klemme 140 schließt und die gesamte Anordnung in den sicheren Zustand überführt.

Die Vorrichtung gemäß Figur 3 wird folgendermaßen betrieben.

Der gesamte extrakorporale Kreislauf einschließlich der Austauscher 112 und 128 wird vor dem Anschließen an den Patienten mit physiologischer Kochsalzlösung gefüllt, was die Benetzung der hydrophilierten Membranen 114 und 130 - wie eingangs erläutert - zur Folge hat. Diese Membranen bilden dann gegenüber den in den Kammern 118 und 134 befindlichen Gasen eine bis zum bubble-point-Druck undurchlässige Sperre und zusätzlich eine flüssige Grenzschicht, die zur Diffusion der auszutauschenden Gase benutzt werden kann.

Nach dem Füllen mit der physiologischen Kochsalzlösung wird der Patient an den extrakorporalen Kreislauf 102 angeschlossen und die Blutpumpe 110 mit einer vorbestimmten Geschwindigkeit in Betrieb gesetzt. Es erfolgt dann die Zuführung von Luft und von Sauerstoff in einem vorbestimmten Verhältnis bzw. geregelt über die Gasförderorgane 146 bzw. 148.

Hierzu wird im Bereich des CO₂-Austauschers 112 der Transmembrandruck mittels der Drucksensoren 150 und 152 ermittelt und die Gasdrosselanordnung 126 so eingestellt, daß der Transmembrandruck in etwa 0 ist. Der CO₂-Austauscher selbst kann durch die Förderrate des Gasförderorgans 148 eingestellt werden, was entweder empirisch oder aber durch die Bestimmung von CO₂ stromauf mittels des CO₂-Sensors 164 oder aber stromab mittels des CO₂-Sensors 166 und eine entsprechende Regelung durch Vergleich des ermittelten Ist-Wertes mit einem voreingestellten Soll-Wert an dem Gasförderorgan 148 erfolgen kann.

Gleichermaßen wird die Oxygenierung in den Oxygenator 128 entweder empirisch oder aber geregelt durchgeführt. Sofern eine Steuerung durchgeführt wird, wird der einzustellende Sauerstoffüberdruck mittels des O₂-Förderorgans 146 in vorbestimmter Weise konstant gehalten, so daß über die Membran 130 hinweg die Oxygenierung von Blut erfolgt. Andererseits kann jedoch aber auch mittels des O₂-Sensors 174 der Sauerstoffgehalt im Blut stromab des Oxygenators 128 ermittelt werden. Der festgestellte Ist-Wert kann dann mit einem voreingestellten Soll-Wert verglichen werden, so daß das O₂-Förderorgan 146 entsprechend geregelt werden kann.

Vorteilhafterweise weist der Oxygenator 128 eine Kleinere Membranoberfläche als der CO₂-Entgaser 112 auf, der üblicherweise bei etwa 1,8 m² liegt, während der Oxygenator üblicherweise eine Membranoberfläche von etwa 1 m² aufweist. Sauerstoffpartialdrücke liegen bei etwa 0,25 bar, während die Sauerstoffsättigung bis zu 99 % beträgt.

Schließlich kann auch stromauf des Oxygenators 128 ein weiterer Sauerstoffsensor 178 vorgesehen sein, der über eine weitere Signalleitung 180 mit der Hauptsteuereinheit 162 verbunden ist. Dieser O₂-Sensor kann zusätzlich oder aber auch ohne den O₂-Sensor 174 zu Steuer- oder Regelungszwecken eingesetzt werden.

## Patentansprüche

1. Vorrichtung (10, 100) zum extrakorporalen Gasaustausch, insbesondere Oxygenieren von Blut mit wenigstens einem Gasaustauscher (18, 118, 128), der durch eine semipermeable Membran (22, 114, 130) in eine Blutkammer (24, 116, 132) und eine Gasaustauschkammer (26, 118, 134) geteilt ist, wobei durch die Blutkammer (26, 116, 132) ein extrakorporaler Blukreislauf (12, 102) und durch die Gasaustauschkammer (26, 118, 134) ein Gasweg (28, 120, 142) gelegt sind, und mit einer in den extrakorporalen Kreislauf (12, 102) eingeschalteten Blutpumpe (20, 82, 110), dadurch gekennzeichnet, daß die Membran (22, 114, 130) hydrophil oder hydrophiliert ist, so daß die Poren mit einer wäßriger Flüssigkeit benetzbar sind und der Gasweg (26, 28, 32; 142, 134) mit sauerstoffhaltigem Gas unter positivem Transmembrandruck von der Gas- zur Blutseite beaufschlagbar ist.

2. Vorrichtung nach Anspruch 1, dadurch gekennzeichnet, daß die Blutpumpe (20, 82) stromauf oder stromab des Gasaustauschers (18) angeordnet ist.

3. Vorrichtung nach Anspruch 1 oder 2, dadurch gekennzeichnet, daß im extrakorporalen Blutkreislauf (102) ein erster Gasaustauscher (112) als CO₂-Austauscher und ein zweiter Gasaustauscher (128) als Oxygenator aufeinanderfolgend angeordnet sind.

4. Vorrichtung nach Anspruch 3, dadurch gekennzeichnet, daß in die Entgasungskammer (116) des ersten Gasaustauschers (112) ein Entgasungsweg (120) gelegt ist, der auf der Zuführungsseite mit einem steuerbaren Luftförderorgan (148) und auf der Abluftseite mit einem veränderbaren Gasdrosselorgan (126) versehen ist und der Transmembrandruck an der Membran (114) des ersten Gasaustauschers (112) mit der Transmembrandruck-Steuereinheit (158) einstellbar ist.

5. Vorrichtung nach Anspruch 3, dadurch gekennzeichnet, daß der zweite Gasaustauscher (128) mit einer Sauerstoffquelle (144) über eine Oxygenierungsleitung (142) verbunden ist, in die ein steuerbares Sauerstoff-Förderorgan (146) eingeschaltet ist.

6. Vorrichtung nach einem der Ansprüche 3 - 5, dadurch gekennzeichnet, daß im extrakorporalen Blutkreislauf (102) wenigstens ein CO₂-Sensor (164, 166) vorgesehen ist, dessen Signal als Ist-Wert auf eine Hauptsteuereinheit (162) geschaltet ist, die den Ist-Wert mit einem Soll-Wert vergleicht und aufgrund des Vergleichsergebnisses den Gasdurchfluß des ersten Gasaustauschers (112) regelt.

7. Vorrichtung nach einem der Ansprüche 3 - 6, dadurch gekennzeichnet, daß im extrakorporalen Blutkreislauf (102) wenigstens ein O₂-Sensor (174) vorgesehen ist, dessen Signal als Ist-Wert auf die Hauptsteuereinheit (162) geschaltet ist, die den Ist-Wert mit einem Soll-Wert vergleicht und aufgrund des Vergleichsergebnisses den am O₂-Förderorgan (146) einstellbaren O₂-Fluß regelt.

8. Vorrichtung nach einem der Ansprüche 1 - 7, dadurch gekennzeichnet, daß die Membran (22, 114, 130) aus einem hydrophilen Material, insbesondere einem Gemisch von Polysulfon und Polyvinylpyrrolidon besteht.

9. Vorrichtung nach einem der Ansprüche 1 - 8, dadurch gekennzeichnet, daß die Membran (22, 114, 130) asymmetrisch ist und eine mikroporöse Hautschicht und eine grobporige Stützschicht aufweist, wobei die mittlere Porengröße in der Hautschicht zwischen 2 und 20 nm liegt und die Starke der Hautschicht höchstens bei mehreren µm liegt.

## Claims

1. An apparatus (10, 100) for extracorporeal gas exchange, particularly for oxygenating blood, with at least one gas exchanger (18, 118, 128) which is divided by a semi-permeable membrane (22, 114, 130) into a blood chamber (24, 116, 132) and a gas exchange chamber (26, 118, 134), an extracorporeal blood circuit (12, 102) being laid through the blood chamber (26, 116, 132) while a gas path (28, 120, 142) passes through the gas exchange chamber, and with a blood pump (20, 82, 110) incorporated into the extracorporeal circuit (12, 102), characterised in that the membrane (22, 114, 130) is hydrophilic or hydrophilised so that the pores are wettable with an aqueous fluid while the gas path (26, 28, 32; 142, 134) is adapted to be exposed to oxygen-containing gas under positive transmembranal pressure from the gas side to the blood side.

2. An apparatus according to claim 1, characterised in that the blood pump (20, 82) is disposed upstream or downstream of the gas exchanger (18).

3. An apparatus according to claim 1 or 2, characterised in that consecutively disposed in the extracorporeal blood circuit (102) are a first gas exchanger (112) as a CO₂ exchanger and a second gas exchanger (128) as an oxygenator.

4. An apparatus according to claim 3, characterised in that incorporated into the degassing chamber (116) of the first gas exchanger (112) is a degassing path (120) which is provided on the feed side with a controllable air supply means (148) and on the air discharge side with a variable gas throttling means (126) and in that the transmembranal pressure at the membrane (114) of the first gas exchanger (112) can be adjusted with the transmembranal pressure control unit (158).

5. An apparatus according to claim 3, characterised in that the second gas exchanger (128) is connected to an oxygen source (144) via an oxygenating line (142) into which a controllable oxygen supply means (146) is incorporated.

6. An apparatus according to one of claims 3 to 5, characterised in that there is in the extracorporeal blood circuit (102) at least one CO₂ sensor (164, 166) from which the signal is switched as an actual value to a main control unit (162) which compares the actual value with a desired value and regulates the through flow of gas in the first gas exchanger (112) on the basis of a result of such comparison.

7. An apparatus according to one of claims 3 to 6, characterised in that there is in the extracorporeal blood circuit (102) at least one O₂ sensor (174) the signal from which is switched as an actual value to the main control unit (162) which compares the actual value with the desired value and regulates the flow of oxygen which is adjustable at the oxygen supply means (148) on a basis of the result of such comparison.

8. An apparatus according to one of claims 1 to 7, characterised in that the membrane (22, 114, 130) consists of a hydrophilic material, in particular a mixture of polysulphone and polyvinyl pyrrolidone.

9. An apparatus according to one of claims 1 to 8, characterised in that the membrane (22, 114, 130) is asymmetrical and has a microporous skin layer and a coarse-pore supporting layer, the mean pore size in the skin layer being between 2 and 20 nm while the thickness of the skin layer is at most around several µm.

## Revendications

1. Dispositif (10, 100) pour l'échange gazeux extracorporel, en particulier pour l'oxygénation du sang avec au moins un échangeur de gaz (18, 118, 128) lequel est divisé par une membrane semi-perméable (22, 114, 130) en une chambre à sang (24, 116, 132) et une chambre d'échange de gaz (26, 118, 134), la chambre à sang (26, 116, 132) est traversée par une circulation de sang extracorporelle (12, 102), et la chambre d'échange de gaz (26, 118, 134) par une conduite de gaz (28, 120, 142), et avec une pompe à sang (20, 82, 110) installée dans la circulation extracorporelle (12, 102), caractérisé en ce que la membrane (22, 114, 130) est hydrophile ou hydrophilisée, de façon telle que les pores soient mouillables par un liquide aqueux et la conduite de gaz (26, 28, 32 ; 142, 134) puisse être chargée par un gaz contenant de l'oxygène sous une pression positive à travers la membrane du côté gaz vers le côté sang.

2. Dispositif selon la revendication 1, caractérisé en ce que la pompe à sang (20, 82) est disposée en aval ou en amont de l'échangeur de gaz (18).

3. Dispositif selon la revendication 1 ou 2, caractérisé en ce que dans la circulation de sang extracorporelle (102) sont disposés successivement un premier échangeur de gaz (112) en tant qu'échangeur de CO₂ et un second échangeur de gaz (128) en tant qu'oxygénateur.

4. Dispositif selon la revendication 3, caractérisé en ce que dans la chambre de dégazage (116) du premier échangeur de gaz (112) est placée une conduite de dégazage (120), laquelle est munie du côté d'alimentation d'un organe de transport d'air pilotable (148) et du côté d'évacuation de l'air, d'un organe d'étranglement de gaz (126) modifiable et la pression à travers la membrane sur la membrane (114) du premier échangeur de gaz (112) est régulable au moyen de l'unité de pilotage de la pression à travers la membrane (158).

5. Dispositif selon la revendication 3, caractérisé en ce que le second échangeur de gaz (128) est relié à une source d'oxygène (144) par l'intermédiaire d'une conduite d'oxygénation (142), dans laquelle est installé un organe de transport d'oxygène pilotable (146).

6. Dispositif selon l'une des revendications 3 à 5, caractérisé en ce que dans la circulation de sang extracorporelle (102), il est prévu au moins un capteur de CO₂ (164, 166), dont le signal en tant que valeur réelle est envoyé à l'unité de pilotage principale (162) laquelle compare la valeur réelle à une la valeur théorique, et sur la base du résultat de comparaison, règule le débit de gaz du premier échangeur de gaz (112).

7. Dispositif selon l'une des revendications 3 à 6, caractérisé en ce que dans la circulation de sang extracorporelle (102), il est prévu au moins un capteur de O₂ (174), dont le signal en tant que valeur réelle est envoyé à l'unité de pilotage principale (162) laquelle compare la valeur réelle à une la valeur théorique, et sur la base du résultat de comparaison, règule le flux de O₂ régulable sur l'organe de transport de O₂.

8. Dispositif selon l'une des revendications 1 à 7, caractérisé en ce que la membrane (22, 114, 130) est constituée d'une matière hydrophile, en particulier d'un mélange de polysulfone et de polyvinylpyrrolidone.

9. Dispositif selon l'une des revendications 1 à 8, caractérisé en ce que la membrane (22, 114, 130) est asymétrique et présente une couche de peau microporeuse et ou couche de support à pores grossiers, la taille moyenne des pores dans la couche de peau étant de 2 à 20 nm et la l'épaisseur de la couche de peau étant de plusieurs µm au maximum.
